# EUROPEAN PATENT APPLICATION

(11) **EP 2 275 814 A1**
(43) Date of publication of application: **19.01.2011**
(21) Application number: 09742624.1
(22) Date of filing: 07.05.2009
(51) Int. Cl.: G01N 33/53, C07K 7/08, C07K 16/18, G01N 33/543, G01N 33/577, C07K 14/47

(54) **A -OLIGOMER MEASUREMENT METHOD**

(30) Priority: 08.05.2008 JP 2008121844
(71) Applicant: Takeda Pharmaceutical Company Limited, Osaka- shi, Osaka 541-0045 (JP)
(72) Inventor: TOKUDA, Takahiko, Kyoto-shi Kyoto 602-0915 (JP); FUKUMOTO, Hiroaki, Osaka-shi Osaka 532-8686 (JP)
(74) Representative: Held, Stephan
(86) International application number: PCT/JP2009/002004
(87) International publication number: WO 2009/136499

(57) **Abstract**

Provided is a measurement method of an Aβ oligomer by the ELISA method, particularly, a method of selectively measuring an Aβ oligomer with a comparatively high molecular weight.

[Solving Means]

A measurement method of an Aβ oligomer including the following steps:
(a) a step of contacting a sample possibly containing an Aβ oligomer with a monoclonal antibody that recognizes a partial peptide consisting of the amino acid sequence shown by SEQ ID NO: 1, which is bound to an insoluble carrier (first antibody) to bind the Aβ oligomer with the first antibody; and
(b) a step of reacting a labeled monoclonal antibody that recognizes a partial peptide consisting of the amino acid sequence shown by SEQ ID NO: 1 (second antibody) with the Aβ oligomer bound to the first antibody in step (a).

## Description

### Technical Field

The present invention relates to a method of measuring an Aβ oligomer.

### Background Art

Alzheimer's disease (AD) is a neurodegenerative disease characterized by progressive loss of cognitive ability, as well as neuropathological characteristics of amyloid deposition, neurofibrillary tangle, loss of nerve cell and the like in the brain.
The role of amyloid β (Aβ) peptide oligomers in neurotic diseases (e.g., Alzheimer's disease, MCI (mild cognitive impairment), Down's syndrome) has been discussed in recent years, and it has been reported that Aβ(1-42) oligomer is the structural basis of pathogenicity, and its formation is the initial pathological event of Alzheimer's disease (Journal of Neurochemistry, 2005, 95, 834-847).
In addition, it has been reported that oligomers of a comparatively high molecular weight, particularly 12 mer, exert a greater influence on memory impairment (Vol 440, Number 7082, 2006, p. 352-357, nature).
Therefore, the development of a measurement method of Aβ oligomer is expected to be effective for the initial diagnosis of Alzheimer's disease, development of a therapeutic drug using Aβ oligomer and the like.
Aβ oligomers can be measured by Western blotting. However, it is difficult to analyze many samples by Western blotting. Therefore, the development of a measurement method of Aβ oligomers based on the ELISA method has been desired.

As an exemplary measurement of Aβ oligomer by the ELISA method, non-patent document 1 describes measurement of Aβ oligomers (2 - 3 mer) by sandwich EIA using antibody 4G8.
Non-patent document 2 describes detection of Aβ oligomers by sandwich EIA using antibody 6E10.
Non-patent document 3 describes detection of Aβ oligomers by sandwich EIA using antibody 2F12 or 6E10.
Non-patent document 4 describes detection of oligomers by sandwich EIA using antibody 2F12.
Non-patent document 5 describes detection of oligomers by sandwich EIA using antibody 2F12.

patent document 1: WO94/17197
non-patent document 1: LeVine et al., Analytical Biochemistry, 335 (2004) p. 81-90
non-patent document 2: Yang et al., The Journal of Biological Chemistry, 2005, Vol. 280, No. 7, p. 5892-5901
non-patent document 3: HOWLETT et al., Biochem. J., 1999, 340, p. 283-289
non-patent document 4: WARD et al., Biochem. J., 2000, 348, p. 137-144
non-patent document 5: Howlett et al., FEBS Letters, 1997, 417, p. 249-251

### Summary of the Invention

### Problems to be Solved by the Invention

Among Aβ oligomers, however, high molecular weight oligomers have been reported to show strong toxicity, and therefore, the development of a measurement method of Aβ oligomers, particularly a method of selectively measuring Aβ oligomer of a comparatively high molecular weight, based on the ELISA method has been desired.

### Means of Solving the Problems

The present inventors have conducted intensive studies and found that Aβ oligomers can be detected, and particularly, Aβ oligomer of a comparatively high molecular weight can be selectively measured, by using, as a first antibody, a monoclonal antibody that recognizes a partial peptide consisting of the amino acid sequence shown by SEQ ID NO: 1 and the first antibody after labeling as a second antibody, and conducted further studies and completed the present invention.

Accordingly, the present invention provides
[1] a method of measuring an Aβ oligomer comprising the following steps:
   (a) a step of contacting a sample possibly containing an Aβ oligomer with a monoclonal antibody that recognizes a partial peptide consisting of the amino acid sequence shown by SEQ ID NO: 1, which is bound to an insoluble carrier (first antibody) to bind the Aβ oligomer with the first antibody; and
   (b) a step of reacting a labeled monoclonal antibody that recognizes a partial peptide consisting of the amino acid sequence shown by SEQ ID NO: 1 (second antibody) with the Aβ oligomer bound to the first antibody in step (a);
[2] the method according to the above-mentioned [1], wherein the monoclonal antibody that recognizes a partial peptide consisting of the amino acid sequence shown by SEQ ID NO: 1 is BAN50a;
[3] the method according to the above-mentioned [1], wherein the Aβ oligomer is of not less than 9 mer;
[4] the method according to the above-mentioned [1], wherein the Aβ oligomer is of not less than 12 mer;
[5] the method according to the above-mentioned [1], wherein the labeled monoclonal antibody (second antibody) is a Fab fragment;
[6] the method according to the above-mentioned [1], wherein the Aβ oligomer is an oligomer of Aβ(1-40), Aβ(1-42), or Aβ(1-43), or an N-terminal shortened form thereof, or a modified form thereof, or a mixture thereof;
[7] an Aβ oligomer measurement kit comprising (a) a monoclonal antibody BAN50a (first antibody) bound to an insoluble carrier; and (b) a labeled monoclonal antibody BAN50a (second antibody);
[8] the measurement kit according to the above-mentioned [7], which is a diagnostic agent for a neurotic disease involving the Aβ oligomer;
[9] a method of screening for a compound that inhibits Aβ oligomer formation, comprising measuring Aβ oligomer by using the measurement kit of the above-mentioned [7];
[10] a method of diagnosing a neurotic disease involving Aβ oligomer, comprising measuring Aβ oligomer by using the measurement kit of the above-mentioned [7];
[11] a method of treating a neurotic disease involving Aβ oligomer, comprising measuring Aβ oligomer by using the measurement kit of the above-mentioned [7];
and the like.

### Effect of the Invention

Using the measurement method of Aβ oligomer of the present invention, an Aβ oligomer of a comparatively high molecular weight can be selectively measured.

### Brief Description of the Drawings

Fig. 1 shows a protein concentration of Aβ42 solution after gel filtration.
Fig. 2 shows the analysis results of each fraction of Aβ42 solution after gel filtration.
Fig. 3 shows the analysis results of inhibition by curcumin of oligomer Aβ formation.
Fig. 4 shows the standard curve of Aβ oligomer based on Aβ42 monomer.
Fig. 5 shows the analysis results of each oligomer Aβ of patients with Alzheimer's disease and healthy human. Embodiment of the Invention

In the present specification, the term "measurement" is used to mean not only measurement of amounts but also mere detection of presence.
In the measurement method of an Aβ oligomer of the present invention, a monoclonal antibody that recognizes a partial peptide consisting of the amino acid sequence shown by SEQ ID NO: 1 is used.
SEQ ID NO: 1 shows the amino acid sequence of Aβ(1-16).
[SEQ ID NO: 1] Asp-Ala-Glu-Phe-Arg-His-Asp-Ser-Gly-Tyr-Glu-Val-His-His-Gln-Lys

As the monoclonal antibody, particularly preferred is a monoclonal antibody BAN50a.
The monoclonal antibody BAN50a is a known antibody described in W094-17197, and can be produced by the method described in the patent document. Alternatively, the monoclonal antibody BAN50a can be produced by a known method and using the hybridoma deposited in the International Patent Organism Depositary (the former Ministry of International Trade and Industry, National Institute of Bioscience and Human-Technology) under an accession number FERM BP4163.

The measurement method of an Aβ oligomer of the present invention includes the following step (a) and step (b). The operation thereof can be performed according to the widely-used ELISA method.
step (a): a step of contacting a sample containing an Aβ oligomer with a monoclonal antibody that recognizes a partial peptide consisting of the amino acid sequence shown by SEQ ID NO: 1, which is bound to an insoluble carrier (first antibody) to bind the Aβ oligomer with the first antibody
step (b): a step of reacting a labeled monoclonal antibody that recognizes a partial peptide consisting of the amino acid sequence shown by SEQ ID NO: 1 (second antibody) with the Aβ oligomer bound to the first antibody in step (a).

The "monoclonal antibody that recognizes a partial peptide consisting of the amino acid sequence shown by SEQ ID NO: 1, which is bound to an insoluble carrier (first antibody)" used in step (a) can be produced according to a widely-used insolubilization (immobilization) method of protein.
For insolubilization (immobilization), physical adsorption may be used, or a method using a chemical bond, which is generally used for insolubilizing or immobilizing protein, enzyme and the like, may be employed. For example, a biotin-(strept)avidin system is preferably used. Examples of the carrier to be used in the present invention include, but are not limited to, insoluble polysaccharides such as agarose, dextran, cellulose and the like; synthetic resins such as polystyrene, polyacrylamide, silicon and the like; and glass.
After linking the aforementioned "monoclonal antibody that recognizes a partial peptide consisting of the amino acid sequence shown by SEQ ID NO: 1" to an insoluble carrier, a blocking treatment may be applied when desired using a commercially available blocking agent.

The "labeled monoclonal antibody that recognizes a partial peptide consisting of the amino acid sequence shown by SEQ ID NO: 1 (second antibody)" used in step (b) can be produced according to a widely-used protein labeling method.
Examples of the labels to be used for the labeling include radioisotope, enzyme (e.g., horse-radish peroxidase (HRP)), fluorescent substance, luminescent substance and the like. Examples of the radioisotope include ¹²⁵I, ¹³¹I, ³H, ¹⁴C and the like. As the enzyme, one which is stable and has high specific activity is preferable, and examples thereof include β-galactosidase, β-glucosidase, alkaline phosphatase, peroxidase and malic acid dehydrogenase. Examples of the fluorescent substance include fluorescamine and fluorescein isothiocyanate. Examples of the luminescent substance include luminol, luminol derivative, luciferin and lucigenin. As the monoclonal antibody to be labeled, IgG, Fab fragment, Fab' fragment or F(ab')₂ fragment (preferably, Fab fragment or Fab' fragment) of a monoclonal antibody can be used. When, for example, horse-radish peroxidase is used for labeling, it can be linked to IgG or Fab fragment of a monoclonal antibody by a conventional method.

A sample (possibly) containing an Aβ oligomer can be prepared, for example, by the following method and the like.

### [Method 1]

Aβ1-42 (e.g., available from PEPTIDE INSTITUTE, INC.) is dissolved in HFIP to 1 mM concentration, dispensed to Eppendorf tubes by 10 µL, and dried by an evaporator. Thereafter, it is cryopreserved at -30°C or lower. The cryopreserved synthetic Aβ peptide is dissolved in DMSO (dimethyl sulfoxide) to 10 µL, further dissolved in a physiological buffer (phosphate buffer; PBS, DMEM (Dulbecco Modified medium), F12 medium and the like) to a final concentration of 10 µM, and incubated in an ice-cold room etc. for a given time (e.g., whole day and night).

### [Method 2]

A biological sample (e.g., part of brain, blood, cerebrospinal fluid of organism) derived from a subject (possibly) affected with a neurotic disease involving Aβ oligomer (e.g., Alzheimer's disease, MCI, Down's syndrome), an elderly subject, an Alzheimer's disease causative gene overexpressing transgenic mouse or the like is extracted with Tris buffer containing, for example, protease inhibitor cocktail, under ice-cooling to give a measurement sample.
The method of the present invention is preferably used for analyzing a sample (possibly) containing an oligomer of preferably high molecule (e.g., about 40kd - 200kd) of not less than 9 mer (particularly preferably oligomer of not less than 12 mer (e.g., 12 mer - 32 mer)).

Step (a) and step (b) may be sequentially or simultaneously performed. Preferably, step (b) is performed after step (a).

After step (b), the second antibody unbound to the Aβ oligomer is washed away.
Then, the signal of the label is measured according to the kind of the label, whereby the Aβ oligomer in the sample is measured.

More specifically, the method of the present invention can be performed, for example, according to the method of the following Example 1.

Using the measurement method of an Aβ oligomer of the present invention, an oligomer of Aβ(1-16) or Aβ containing a part thereof (e.g., Aβ(3-16)), or a modified form thereof or a mixture thereof can be measured. Examples of the Aβ include Aβ(1-40), Aβ(1-42), Aβ(1-43), and N-terminal shortened forms thereof (e.g., Aβ(3-42), Aβ(3-42), and Aβ(3-43)).

Examples of the amino acid sequences of Aβ(1-40), Aβ(1-42), and Aβ(1-43), Aβ(3-40), Aβ(3-42), and Aβ(3-43) are shown below.
[Aβ(1-40)] SEQ ID NO: 2 
Asp-Ala-Glu-Phe-Arg-His-Asp-Ser-Gly-Tyr-Glu-Val-His-His-Gln-
Lys-Leu-Val-Phe-Phe-Ala-Glu-Asp-Val-Gly-Ser-Asn-Lys-Gly-Ala-
Ile-Ile-Gly-Leu-Met-Val-Gly-Gly-Val-Val
 [Aβ(1-42)] SEQ ID NO: 3 
-
-

 [Aβ(1-43)] SEQ ID NO: 4 
Asp-Ala-Glu-Phe-Arg-His-Asp-Ser-Gly-Tyr-Glu-Val-His-His-Gln-
Lys-Leu-Val-Phe-Phe-Ala-Glu-Asp-Val-Gly-Ser-Asn-Lys-Gly-Ala-
Ile-Ile-Gly-Leu-Met-Val-Gly-Gly-Val-Val-Ile-Ala-Thr
 [Aβ(3-40)] SEQ ID NO: 5 
Glu-Phe-Arg-His-Asp-Ser-Gly-Tyr-Glu-Val-His-His-Gln-Lys-Leu-
Val-Phe-Phe-Ala-Glu-Asp-Val-Gly-Ser-Asn-Lys-Gly-Ala-Ile-Ile-
Gly-Leu-Met-Val-Gly-Gly-Val-Val
 [Aβ(3-42)] SEQ ID NO: 6 
Glu-Phe-Arg-His-Asp-Ser-Gly-Tyr-Glu-Val-His-His-Gln-Lys-Leu-
Val-Phe-Phe-Ala-Glu-Asp-Val-Gly-Ser-Asn-Lys-Gly-Ala-Ile-Ile-
Gly-Leu-Met-Val-Gly-Gly-Val-Val-Ile-Ala
 [Aβ(3-43)] SEQ ID NO: 7

Glu-Phe-Arg-His-Asp-Ser-Gly-Tyr-Glu-Val-His-His-Gln-Lys-Leu-
Val-Phe-Phe-Ala-Glu-Asp-Val-Gly-Ser-Asn-Lys-Gly-Ala-Ile-Ile-
Gly-Leu-Met-Val-Gly-Gly-Val-Val-Ile-Ala-Thr

As the aforementioned modified form, N-terminal modified forms such as pyroglutamate and the like, amino acid substituted form and the like can be mentioned. Such modification can be performed by a method conventionally used by those of ordinary skill in the art. In addition, such modified form can also be obtained from a natural sample.

Using the measurement method of an Aβ oligomer of the present invention, 2 - 6 mer forms are not substantially measured, and an oligomer of high molecule (about 40kd - 200kd) of not less than 9 mer (particularly preferably oligomer of not less than 12 mer (e.g., 12 mer - 32 mer)) can be measured selectively.

The present invention also provides an Aβ oligomer measurement kit containing
(a) a monoclonal antibody BAN50a bound to an insoluble carrier (first antibody); and
(b) a labeled monoclonal antibody BAN50a (second antibody).

In addition to
(a) a monoclonal antibody BAN50a bound to an insoluble carrier (first antibody); and
(b) a labeled monoclonal antibody BAN50a (second antibody), the kit may be provided with various reagents and equipment necessary for the ELISA method.
The kit can be used for performing the measurement method of the present invention, and can selectively measure an oligomer of high molecule (about 40kd - 200kd) of not less than 9 mer (particularly preferably oligomer of not less than 12 mer (e.g., 12 mer - 32 mer)).

A compound that inhibits formation of an Aβ oligomer can be searched (screened) for by measuring the Aβ oligomer by using the measurement method of the present invention or the measurement kit of the present invention.
For example, a compound that inhibits formation of an Aβ oligomer can be searched (screened) for by
(1) adding a test compound to a culture medium of a primary nerve cell, measuring the amount of an Aβ oligomer in the culture supernatant by the measurement method of the present invention or the measurement kit of the present invention, and selecting a compound having an activity to reduce the amount, or
(2) measuring the intracerebral amount of an Aβ oligomer of an animal administered with the test compound by the measurement method of the present invention or the measurement kit of the present invention, and selecting a compound having an activity to reduce the amount.

More specifically, the method can be performed by, for example, the method of the following Example 2.

In addition, analysis of neurotic diseases (e.g., Alzheimer's disease, MCI, Down's syndrome) involving Aβ oligomers is enabled by measuring the Aβ oligomer by using the measurement method of the present invention or the measurement kit of the present invention. The present invention is particularly preferably used for Alzheimer's disease.
For example, neurotic diseases involving Aβ oligomers can be diagnosed by measuring the Aβ oligomer by using the measurement method of the present invention or the measurement kit of the present invention. That is, the measurement kit of the present invention can be used as a diagnostic agent of neurotic diseases involving Aβ oligomers.
Specifically, for example, the presence or absence of neurotic diseases involving Aβ oligomers, and the level of pathological progression thereof can be diagnosed by measuring the amount of the Aβ oligomer in the cerebrospinal fluid of the subject by using the measurement method of the present invention or the measurement kit of the present invention.

In addition, the efficacy of a medicament for the prophylaxis or treatment of neurotic diseases involving Aβ oligomers can be evaluated by measuring the amount of the Aβ oligomer in the cerebrospinal fluid etc. of the patients affected with the disease and administered with the medicament, by using the measurement method of the present invention or the measurement kit of the present invention. Examples of the medicament include Aβ aggregation inhibitors (e.g., Alzhemed), Aβ production reducers (e.g., Flurizan, LY-450139), and anti-Aβ (including oligomer form) antibody.
That is, the measurement method of the present invention, and the measurement kit of the present invention can be used for monitoring a treatment effect of a medicament for the prophylaxis or treatment of neurotic diseases involving Aβ oligomers, or evaluation of investigational agents.

Even patients with Alzheimer's disease show great variation in the amount of intracerebral Aβ oligomers. While patients with higher amounts of Aβ oligomer in the cerebrospinal fluid are assumed to show greater amounts of intracerebral Aβ oligomers, patients more suitable for clinical trial of a medicament for the prophylaxis or treatment of neurotic diseases involving Aβ oligomers (e.g., Aβ oligomer formation inhibitor) can be selected by measuring the amount of the Aβ oligomer in the cerebrospinal fluid etc. of the subjects by using the measurement method of the present invention or the measurement kit of the present invention.
Moreover, tailor-made treatments using more suitable treatment methods for patients become available by measuring the amount of the Aβ oligomer in the cerebrospinal fluid etc. of the patients affected with neurotic diseases involving Aβ oligomers, by using the measurement method of the present invention or the measurement kit of the present invention.

The presence or absence of neurotic diseases (e.g., Alzheimer's disease, MCI, Down's syndrome), and the level of pathological progression thereof can be diagnosed by combining the measurement method or measurement kit of the present invention with a measurement method of tau, phosphorylated tau, sAPPα, APPβ or the like, or a measurement method of other Aβ (Aβ1-40, Aβ1-42, or N-terminal shortened form thereof) . Examples

The present invention is explained in more detail in the following by referring to Examples and Experimental Examples, which are not to be construed as limitative.

Abbreviations in the following Examples are as described below.

### HRP: horseradish peroxidase

In the following Examples, ELISA was performed using the following buffers.
[buffer]
coating buffer A
(0.1 M carbonate buffer, pH 9.6)
blocking buffer B
(25% Block Ace in PBS)
buffer C
(pH 7.0, 20 mM phosphate buffer, 10% Block Ace, 0.2% BSA, 0.05% Merthiolate (TM) Na (thimerosal), 400 mM NaCl, 0.076% CHAPS, 2 mM Na₂EDTA, after heat treatment at 56°C for 30 min, preserved in cold room)

### Example 1

ELISA using BAN50a and HRP labeled BAN50a (quantification of oligomer Aβ) was performed by the following steps.
(1) BAN50a antibody was dissolved in coating buffer A to a concentration of 10 µg/ml (75 µl/well), added to an EIA96 well plate (high maximum binding clear plate, Greiner), and incubated in a refrigerating room (about 4°C) for a whole day and night.
(2) After washing 3 times with PBS (calcium, magnesium-free; hereinafter sometimes to be abbreviated as PBS(-)), blocking buffer B was added to 100 µL/well and the plate was stored.
(3) On the day of experiment, blocking buffer B was discarded and the plate was washed twice with PBS(-), and 25 µl of buffer C was added.
(4) A solution of standard monomer Aβ1-42 [Aβ1-42 TFA salt (PEPTIDE INSTITUTE, INC.) was dissolved in DMSO to 1 mM, further diluted with F12 medium (GIBCO) to 10 µm, dispensed by 1 ml, stood in a cold room (about -4°C) for a whole day and night, and the solution (containing an oligomer formed therein) was diluted with buffer C to give a 1 µM solution based on a standard monomer concentration, dispensed and preserved at -80°C. When in use, the dispensed 1 µM solution was diluted with a sample buffer to give a 2-fold dilution series (10 - 0.0097 nM based on Aβ1-42 concentration) and used as a standard.] was processed with buffer C to give 2-fold dilution series from 100 nM to 0.390625 nM as Aβ monomer corresponding concentration. The sample was diluted to a suitable concentration, confirmed to fall within the standard curve and added to a plate at 100 µL/well.
(5) The sample was incubated in a refrigerating room (about 4°C) for a whole day and night.
(6) The plate was washed 4 times with PBS(-).
(7) As a secondary antibody, HRP-labeled BAN50a (Fab'-HRP for measurement of biological sample (i.e., in vivo)) was 5000-fold diluted with and dissolved in buffer D and added to 75 µL/well. The secondary antibody was produced by a professional manufacturer (Wako Pure Chemical Industries, Ltd.).
(8) The plate was incubated at room temperature for 3 hr or longer.
(9) The plate was washed 6 times with PBS(-).
(10) TMB substrate (Kirkegaard&Perry) was added to each plate at 75 µl/well, and incubated at room temperature.
(11) Thereafter, 1 M phosphoric acid solution (75 µl/well) was added to the reacted substrate solution to terminate the reaction, and OD₄₅₀ was measured by a multi-label counter (PerkinElmer).

### Example 2

An influence of test compound on oligomer formation was examined by the following method.
(1) Aβ1-42 was dissolved in DMSO to 300 µM.
(2) A reaction mixture with a suitable composition was prepared and placed in a tube.
[Example composition of the reaction mixture (Aβ1-42 concentration of final reaction solution of Aβ1-42 was 3 µM, compound concentration was 3 µM)]

| | |
|---|---|
| Aβ1-42 (300 µM) | 1 µL |
| Curcumin solution (300 µM) | 1 µL |
| F12 medium | 98 µL |
| Total | 100µL |

(3) The tube was incubated at 4°C for a whole day and night.
(4) The reaction mixture was diluted 100-fold with buffer C (mentioned above), and measured by the aforementioned ELISA.
(5) The inhibitory rate was calculated with that of the vehicle group as 100%.

### Experimental Example 1

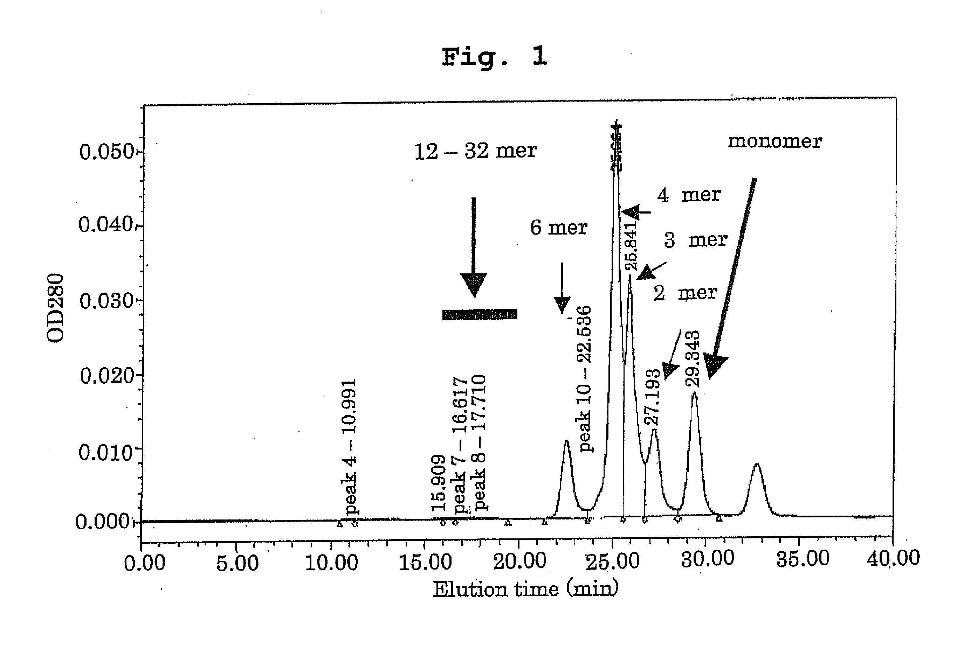

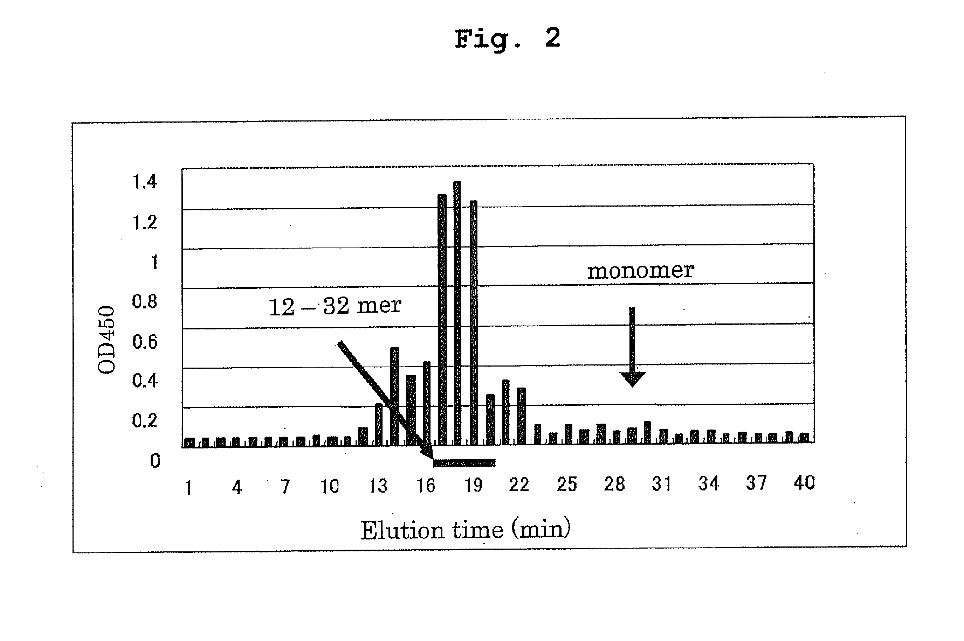

The molecular species of the oligomer Aβ recognized by the ELISA system of Example 1 was identified. Aβ1-42 TFA salt (PEPTIDE INSTITUTE, INC.) was dissolved in DMSO to 1 mM, further dissolved in F-12 medium to 10 µM, and incubated at 4°C for a whole day and night. Then, the sample was pretreated using a filtration membrane (0.45 µm) and 300 µL was applied to a gel filtration column. For gel filtration, elution was performed using the Alliance system of Waters with a combination of two Biosuites 450 and 125, PBS(-) as an elution buffer at a rate of 1 mL/min in an ice-cold room. For calibration of the molecular weight, two marker sets of low molecular weight and high molecular weight of Amersham were used. Calibration was performed by plotting by Excel (Microsoft) with Log (molecular weight) in the Y axis and elution time in the X-axis. The amount of protein was monitored at OD=280 nm (Fig. 1). In addition, a sample solution fractionated by elution was diluted 10-fold or more with Buffer C and analyzed using the ELISA system of Example 1. As a result, monomer and comparatively low molecular oligomer (2 mer - 6 mer) were not detected by the ELISA system of Example 1, and a high molecular oligomer (12 mer - 32 mer) eluted from 17 min to 19 min was detected with high sensitivity (Fig. 2).

### Experimental Example 2

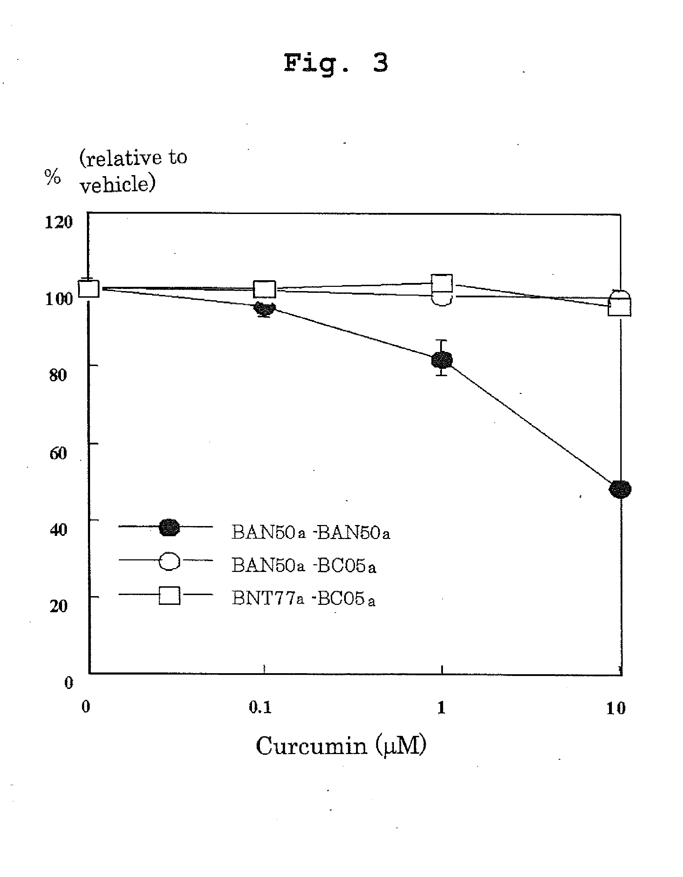

According to Example 2, inhibition of formation of high molecular oligomer Aβ by curcumin known to inhibit oligomer Aβ formation was analyzed. Curcumin with various concentrations was added to Aβ42 solutions, an influence of curcumin on oligomer Aβ formation was examined by the ELISA of Example 1. As a result, the signal of oligomer Aβ specifically decreased. The results are shown in Fig. 2. As a control, the results of similar analysis of BAN50a-HRP-labeled BC05a and BNT77a-HRP-labeled BC05a, that recognize monomers, by ELISA (performed according to the method described in Asami-Odaka A, Ishibashi Y, Kikuchi T, Kitada C, Suzuki N., Long amyloid beta-protein secreted from wild-type human neuroblastoma IMR-32 cells. Biochemistry. 1995 Aug 15; 34(32): 10272-8.) are also shown in Fig. 3.

### Experimental Example 3

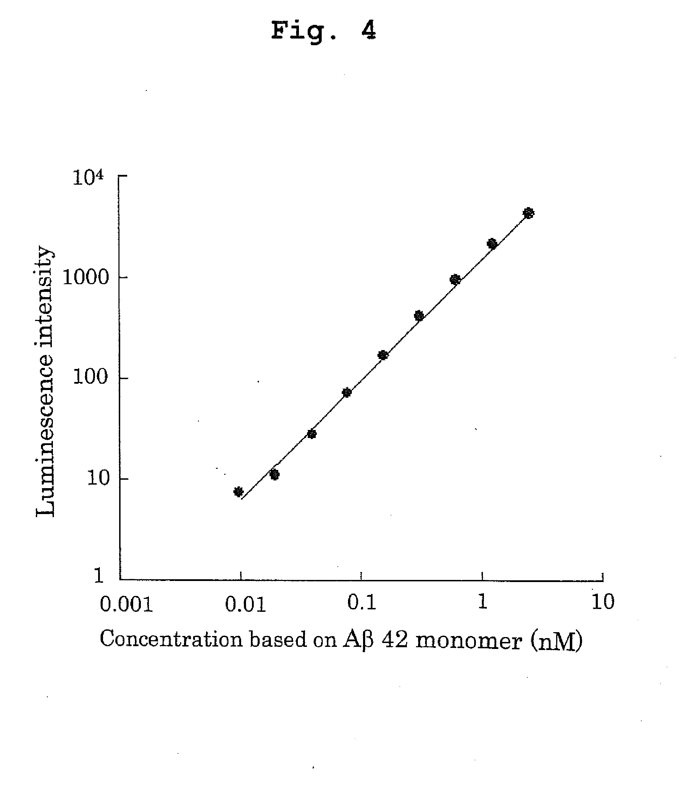

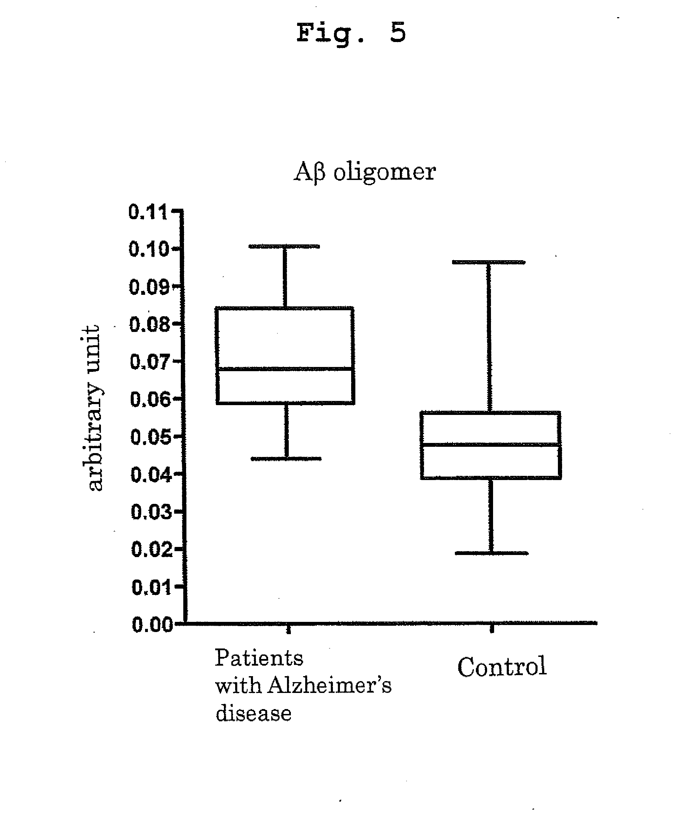

BAN50a antibody was dissolved in 0.1 M carbonate buffer (pH 9.6) to a concentration of 10 µg/ml, added to a 96-well polystyrene plate (FLUOTRAC(TM) 600, Greiner Bio-One) (75 µl/well) and incubated at 4°C for 24 hr. The plate was washed 3 times with PBS(-) (Ca, Mg-free PBS, DULBECCO'S PBS, Dainippon Sumitomo Pharma Co., Ltd., Laboratory Products Division), a blocking buffer (PBS(-)/0.8% BlockAce (Dainippon Sumitomo Pharma Co., Ltd., Laboratory Products Division)/0.01% Merthiolate Na) was added to 100 µl/well and the plate was stored at 4°C. On the day of experiment, the blocking buffer was discarded, washed twice with PBS(-), and 25 µl of a sample buffer (0.02M phosphate buffer/0.4% BlockAce/0.2% BSA/0.05% Merthiolate Na/0.4M NaCl/0.076% CHAPS/2 mM Na₂EDTA) was added to each well. The cerebrospinal fluid of a patient with Alzheimer's disease (healthy human cerebrospinal fluid as control) was diluted 2-fold with a sample buffer, having a 2-fold concentration and added with a protease inhibitor (complete, Mini, Roche Diagnostics) (1 tablet/5 ml), and added to each well by 100 µl. In addition, Aβ1-42 TFA salt (PEPTIDE INSTITUTE, INC.) was dissolved in DMSO to 1 mM, further diluted with F12 medium (GIBCO) to 10 µM, dispensed by 1 ml, stood in a cold room (about -4°C) for a whole day and night, diluted with buffer C to give 1 µM solution, dispensed and preserved at -80°C. When in use, the dispensed 1 µM solution was diluted with a sample buffer to give a 2-fold dilution series (2.5 - 0.0097 nM based on Aβ1-42 monomer concentration) and used as a standard. That is, Aβ42 oligomer present in Aβ1-42 solution (2-fold dilution series, 100 µl/well) prepared by the completely same procedure in each time of experiment was used as the standard. A sample diluted with buffer C and the standard solution were added to a plate. The plate was stored at 4°C for 24 hr, and washed 4 times with PBS(-), HRP labeled BAN50a antibody (1000- to 5000-fold diluted solution) was added at 100 µl/well and the plate was incubated at room temperature for 3 hr. After incubation, the plate was washed 6 times with PBS(-), and a chemiluminescent substrate (SuperSignal ELISA Femto, PIERCE) was added at 100 µl/well, and the luminescence intensity was quantified by a luminometer (SpectraMax L (trade name), Molecular Devices). The results of the standard curve are shown in Fig. 4, and the results of cerebrospinal fluid sample measurement are shown in Fig. 5. As shown in Fig. 5, the concentration of Aβ oligomer in the cerebrospinal fluids of patients with Alzheimer's disease and healthy human could be significantly distinguished by this method.

## Claims

1. A method of measuring an Aβ oligomer comprising the following steps:
(a) a step of contacting a sample possibly containing an Aβ oligomer with a monoclonal antibody that recognizes a partial peptide consisting of the amino acid sequence shown by SEQ ID NO: 1, which is bound to an insoluble carrier (first antibody) to bind the Aβ oligomer with the first antibody; and
(b) a step of reacting a labeled monoclonal antibody that recognizes a partial peptide consisting of the amino acid sequence shown by SEQ ID NO: 1 (second antibody) with the Aβ oligomer bound to the first antibody in step (a).

2. The method according to claim 1, wherein the monoclonal antibody that recognizes a partial peptide consisting of the amino acid sequence shown by SEQ ID NO: 1 is BAN50a.

3. The method according to claim 1, wherein the Aβ oligomer is of not less than 9 mer.

4. The method according to claim 1, wherein the Aβ oligomer is of not less than 12 mer.

5. The method according to claim 1, wherein the labeled monoclonal antibody (second antibody) is a Fab fragment.

6. The method according to claim 1, wherein the Aβ oligomer is an oligomer of Aβ(1-40), Aβ(1-42), or Aβ(1-43), or an N-terminal shortened form thereof, or a modified form thereof, or a mixture thereof.

7. An Aβ oligomer measurement kit comprising (a) a monoclonal antibody BAN50a (first antibody) bound to an insoluble carrier; and
(b) a labeled monoclonal antibody BAN50a (second antibody).

8. The measurement kit according to claim 7, which is a diagnostic agent for a neurotic disease involving the Aβ oligomer.

9. A method of screening for a compound that inhibits Aβ oligomer formation, comprising measuring Aβ oligomer by using the measurement kit of claim 7.

10. A method of diagnosing a neurotic disease involving Aβ oligomer, comprising measuring Aβ oligomer by using the measurement kit of claim 7.

11. A method of treating a neurotic disease involving Aβ oligomer, comprising measuring Aβ oligomer by using the measurement kit of claim 7.


SEQUENCE LISTING

<110>  Takeda Pharmaceutical Company Limited

<120>  A method for determining beta amyloid oligomers

<130>  TKDW0080132P

<150>  JP 2008-121844
<151>  2008-05-08

<160>  7

<170>  PatentIn version 3.5

<210>  1
<211>  16
<212>  PRT
<213>  Homo sapiens

<400>  1

Asp Ala Glu Phe Arg His Asp Ser Gly Tyr Glu Val His His Gln Lys
1               5                   10                  15


<210>  2
<211>  41
<212>  PRT
<213>  Homo sapiens

<400>  2

Asp Ala Glu Phe Arg His Asp Ser Gly Tyr Glu Val His His Gln Lys
1               5                   10                  15


Leu Val Phe Phe Ala Glu Asp Val Gly Ser Asn Lys Gly Ala Ile Ile
            20                  25                  30


Gly Leu Met Val Gly Gly Val Val Ile
        35                  40


<210>  3
<211>  42
<212>  PRT
<213>  Homo sapiens

<400>  3


<210>  4
<211>  43
<212>  PRT
<213>  Homo sapiens

<400>  4

Asp Ala Glu Phe Arg His Asp Ser Gly Tyr Glu Val His His Gln Lys
1               5                   10                  15

Leu Val Phe Phe Ala Glu Asp Val Gly Ser Asn Lys Gly Ala Ile Ile
            20                  25                  30

Gly Leu Met Val Gly Gly Val Val Ile Ala Thr
        35                  40

<210>  5
<211>  38
<212>  PRT
<213>  Homo sapiens

<400>  5

Glu Phe Arg His Asp Ser Gly Tyr Glu Val His His Gln Lys Leu Val
1               5                   10                  15

Phe Phe Ala Glu Asp Val Gly Ser Asn Lys Gly Ala Ile Ile Gly Leu
            20                  25                  30

Met Val Gly Gly Val Val
        35

<210>  6
<211>  40
<212>  PRT
<213>  Homo sapiens

<400>  6

Glu Phe Arg His Asp Ser Gly Tyr Glu Val His His Gln Lys Leu Val
1               5                   10                  15

Phe Phe Ala Glu Asp Val Gly Ser Asn Lys Gly Ala Ile Ile Gly Leu
            20                  25                  30

Met Val Gly Gly Val Val Ile Ala
        35                  40

<210>  7
<211>  41
<212>  PRT
<213>  Homo sapiens

<400>  7

Glu Phe Arg His Asp Ser Gly Tyr Glu Val His His Gln Lys Leu Val
1               5                   10                  15

Phe Phe Ala Glu Asp Val Gly Ser Asn Lys Gly Ala Ile Ile Gly Leu
            20                  25                  30

Met Val Gly Gly Val Val Ile Ala Thr
        35                  40